# EUROPEAN PATENT SPECIFICATION

(11) **EP 2 874 669 B1**
(45) Date of publication and mention of the grant of the patent: **06.09.2017**
(21) Application number: 13774531.1
(22) Date of filing: 18.07.2013
(51) Int. Cl.: A61L 2/28

(54) **METHOD FOR CONTROLLING STERILITY OF A STERILIZED MEDICAL DEVICE AND STERILIZATION MODULE FOR CARRYING OUT SAID METHOD**
VERFAHREN ZUR KONTROLLE DER STERILITÄT EINER STERILISIERTEN MEDIZINISCHEN VORRICHTUNG UND STERILISATIONSMODUL ZUR DURCHFÜHRUNG DES VERFAHRENS
PROCÉDÉ DE CONTRÔLE DE LA STÉRILITÉ D'UN DISPOSITIF MÉDICAL STÉRILISÉ ET MODULE DE STÉRILISATION POUR EXÉCUTER LEDIT PROCÉDÉ

(30) Priority: 18.07.2012 SI 201200235
(43) Date of publication of application: 27.05.2015
(73) Proprietor: Kozin, Peter, 1000 Ljubljana (SI)
(72) Inventor: Kozin, Peter, 1000 Ljubljana (SI)
(74) Representative: Golmajer Zima, Marjanca
(86) International application number: PCT/SI2013/000045
(87) International publication number: WO 2014/014424

(56) References cited:
- EP-A1- 0 979 658
- EP-A1- 1 230 936
- WO-A1-00/06211

## Description

The invention relates to a method for controlling sterility of a sterilized medical device after high-temperature steam sterilization, wherein the medical device is placed close to a sterilization module, they are both wrapped by a sterilization wrapper and put into an autoclave to carry out sterilization. The invention also relates to a sterilization module intended for controlling sterility of a sterilized medical device and for carrying out said method. The module is provided with a communication circuit. The sterilization module is foreseen for a routine control of efficiency of sterilization and is reusable.

Controlling of efficiency of sterilization of medical devices, e.g. surgical instruments, by means of high-temperature steam in an autoclave is known. Said controlling is based on determination of colour of an indicator label, which is arranged on the medical device prior to sterilization and they are both wrapped by a sterilization wrapper. The indicator label is disposable. Its colour indicates the extent, to which a chemical reaction therein occured. Said extent is characteristic of a period of maintenance at a prescribed high temperature. On the one hand, uncertainty of assessed efficiency of sterilizing is quite high, on the other hand, the sterilization wrapper needs to be unwrapped before assessing the status of the indicator label. This is only done directly prior to use of a medical device. Due to the mentioned drawbacks, such controlling of efficiency of sterilization only has a limited application in sterilization of medical devices.

EP1230936 A1 discloses a device for determining sterilization conditions, comprising a reference chamber which can be placed in a sterilization space, which chamber is provided with a transducer, with the aid of which the degree of saturation of the sterilant adjacent the transducer can be determined and which chamber is provided with an opening having a geometry such that a sterilant, with respect to the sterilization space, has relatively difficult access to the transducer, characterized in that the transducer can produce an electronic signal from which the sterilization conditions adjacent the transducer can be derived, the device further comprises a temperature sensor for measuring the temperature in the sterilization space, a pressure sensor for measuring the pressure in the sterilization space and an electronic computing unit, for deriving the sterilization conditions adjacent the transducer from the measured temperature and pressure.

Patent US 7,122,150 B2 discloses an electronic reader, which is wrapped into a sterilization wrapper together with a medical device. A disposable label is used as a sterilization indicator. It is inserted into said reader and the colour of the illuminated label after sterilization is read out. The read-out value is evaluated in an interpretation circuit of the reader. The reader determines efficiency of the performed sterilization and the evaluation result is wirelessly signalled to an interrogator by means of a reader communication circuit. Indeed the efficiency of the performed sterilization is determined before the sterilization wrapper is removed and the reader is reusable as well, but said very high uncertainty of measurement still remains due to integral sensing of development during sterilization only by the colour of the indicator label as the only recorded parameter.

Good practice in the field of hospital sterilization of medical devices as well as standards ISO EN 1 1607 and ISO EN 17665 require that sterilization should be carried out in a controlled, reproducible and traceable way.

The technical problem to be solved by the present invention is to propose a method, which should provide for automatical determination of efficiency of performed sterilization by logging a time history of the temperature of a medical device as well as the pressure in its environment during sterilization and, when a sterilization package is unwrapped directly prior to using a medical device, information on sterilization efficiency determined in said way and on potential sterility expiration date should be conveyed to a user and, additionally, an operator of an sterilization machine should be able to make an ex post analysis of time history from the beginning of the sterilization of the medical device onwards. A further task of the invention is to propose a module for carrying said method.

The technical problem of the invention is solved by the method of the invention intended for controlling sterility of a sterilized medical device as characterized by the features of claim 1 and by the sterilization module for controlling sterility of a sterilized medical device as characterized by the features of claim 10. Dependent claims characterize variants of their embodiments.

The technical solution as proposed by the invention primarily stands out for the following advantages. The sterility status of a uniquely identified medical device is unambiguously and simply presented to a user by the fact that a single light source on the sterilization module lights up. The sterilization module is suitable for a routine control of efficiency of sterilization and is reusable.

Immediately after sterilization is over, its efficiency is assessed on the basis of time history of temperature and pressure that were electronically acquired by the sterilization module. An analysis of the values of temperature, pressure and other physical quantities during sterilization is made possible as well.

The invention will now be explained in more detail by way of a description of an embodiment and variants of the method of the invention for controlling sterility of a sterilized medical device as well as a sterilization module of the invention and with reference to the accompanying drawing representing in
Fig. 1 a block diagram of the sterilization module of the invention for controlling sterility of a sterilized medical device, and
Fig. 2 a sterilization package comprising the sterilization module of the invention and the medical device as well as an interrogator and a computer for carrying out further advantageous processing steps after sterilization is completed.

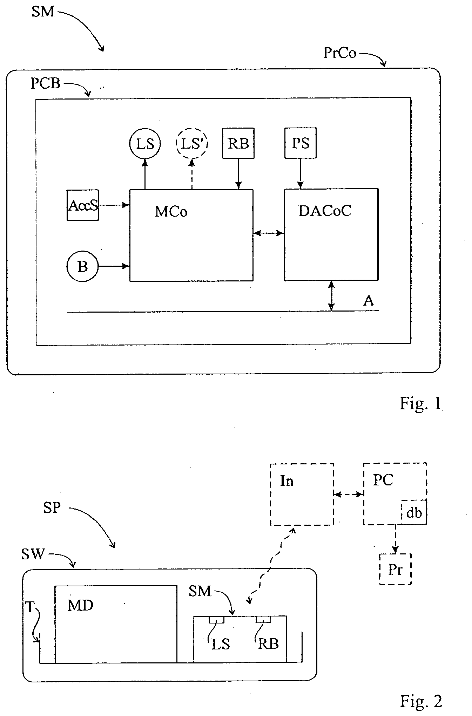

A method of the invention for controlling sterility of a sterilized medical device MD is foreseen to be used in high-temperature steam sterilization. In a known way, the medical device MD is placed on a tray T close to a sterilization module SM, and they are wrapped by a sterilization wrapper SW (Fig. 2). A sterilization package SP is put into an autoclave to carry out steam sterilization.

Prior to first use, the sterilization module SM is labelled by entering its identification mark into its memory.

According to the invention, the sterilization module SM is reset before sterilization is initiated. The sterilization module SM thus gets initialized and activated. Before the sterilization module SM together with the medical device MD are wrapped by the sterilization wrapper SW, a reset button RB is pressed, for example, to reset the sterilization module SM (Fig. 1).

According to the invention, the sterilization module SM logs a time history of the temperature within the sterilization package SP, i.e. the temperature of the medical device MD. During the sterilization the sterilization module SM logs a time history of pressure within the sterilization package SP as well.

According to the invention upon completion of sterilization, the sterilization module SM automatically processes the acquired data and assesses the efficiency of sterilization.

According to the invention, the sterilization module SM shifts from a normal mode to an alarm mode if it is determined during said data processing that the sterilization was inefficient or that sterility expiration date of the medical device MD expired later.

The sterility expiration date is calculated according to the standards in force, namely from completion of sterilization onwards. During sterilization, the sterilization module SM therefore detects a drop in temperature below the prescribed temperature value as sterilization completion.

According to the invention, the sterilization module SM keeps transmitting a signal on its mode for several seconds upon each jerk.

It is accordingly proposed by the invention that, in an advantageous way, a single light source LS on the sterilization module SM signals a basic signal on the mode of the sterilization module SM.

The sterilization module SM is in a normal mode if the medical device MD is sterile, which means that the sterilization was efficient and the sterility expiration date of the medical device MD is not yet over. The sterilization module SM is in an alarm mode if sterilization was inefficient or the sterility expiration date of the medical device MD is over after an efficient sterilization.

As a variant embodiment of the method of the invention it is proposed that, by their way of turning on, light sources LS, LS', ... - variously coloured light emitting diodes may be used - on the sterilization module SM signal data on efficiency of performed sterilization and on sterility expiration date of the medical device MD non being over as well as on other characteristic features of the performed sterilization.

In an advantageous way, the sterilization module SM automatically signals data on sterilization and sterility of the medical device MD to an interrogator In when it is moved to its close proximity. Then the interrogator In transmits said data to a personal computer PC. A printer Pr can then print the data from a computer database db onto a partly two-layered label. The first part of said label is stuck onto the sterilization wrapper SW comprising the sterilized medical device MD and the sterilization module SM, and the second part of the label is stuck onto a patient file directly prior to use of the sterilized medical device MD.

The invention further proposes, a sterilization module SM for controlling sterility of a sterilized medical device MD. The sterilization module SM is equipped for radio communication.

According to the invention, the sterilization module SM is provided with a reset circuit that initializes and activates the sterilization module SM before sterilization is initiated. The sterilization module SM is for instance provided with a reset button RB for resetting that is used before the sterilization module SM together with the medical device MD are wrapped by the sterilization wrapper SW.

The sterilization module SM of the invention is further provided with a data acquisition and communication circuit DACoC. Said circuit, e.g. IDS-SL900A, comprises at least one temperature sensor, a pressure sensor PS may be connected thereto. The data acquisition and communication circuit DACoC is connected to an antenna A.

According to the invention, the sterilization module SM is provided with a microcontroller MCo connected to the data acquisition and communication circuit DACoC. The microcontroller MCo automatically processes the acquired data immediately after completion of sterilization and maintains the sterilization module SM in a normal operation mode or shifts it to an alarm mode depending on efficiency of the performed sterilization and/or on the expired sterility date of the medical device MD.

The sterilization module SM of the invention is further provided with an acceleration sensor AccS connected to an input of the microcontroller MCo. Upon each jerk of the sterilization module SM the acceleration sensor AccS generates a signal that triggers the microcontroller MCo to keep signalling the mode of the sterilization module SM for several seconds.

The sterilization module SM is also provided with a light source LS connected to an input of the microcontroller MCo. Depending on the sterilization module mode, the light source LS signals either a signal of sterility of the medical device MD or a signal of the alarm mode. The light source LS keeps transmitting said signal for several seconds. The sterilization module SM may be provided with several light sources LS, LS', ... , which may be variously coloured and are connected to inputs of the microcontroller MCo. By their way of turning on, they signal the data on the efficiency of the performed sterilization and/or on the sterility expiration date of the medical device MD being over as well as on other characteristic features of the performed sterilization.

Light emitting diodes are used as light sources LS, LS', ...

A battery B supplies a circuit of the sterilization module SM. Said circuit is fabricated on a printed circuit board PCB having dimensions such as 4 cm x 9 cm x 1 cm. The printed circuit board PCB is coated by a thin polymer protective coating PcCo. All components of the sterilization module SM are resistant to high temperature existing during steam sterilization.

The database db of data on the latest sterilization as well as on previous sterilizations performed by said sterilization module SM is created in the personal computer PC. The personal computer PC is connected through an interrogator In with the sterilization module SM, wherefrom it receives the acquired data. The data for printing a partly two-layered label for the sterilized medical device MD after sterilization are acquired from said database db. One part of said label is stuck onto the sterilization package SP, which comprises the medical device MD and the sterilization module SM, and second part thereof is stuck onto a patient file.

## Claims

1. A method for controlling sterility of a sterilized medical device after a high-temperature steam sterilization,
wherein the medical device is placed close to a sterilization module, they are both wrapped by a sterilization wrapper and
put into an autoclave to carry out sterilization, wherein the sterilization module is reset before sterilization is initiated, that the sterilization module logs a time history of
its own temperature and pressure in its own environment
during sterilization,
that the sterilization module assesses the efficiency of sterilization by processing the acquired data,
that the sterilization module shifts from a normal mode to an alarm mode after an inefficient sterilization and/or
after a sterility expiration date of the medical device is over
and that the sterilization module which is provided with an acceleration sensor (AccS) connected to an input of the microcontroller (MCo) signals its mode upon each jerk.

2. The method as recited in claim 1, **characterized in that** the sterilization module is reset by pressing a reset button before the sterilization module together with the medical device is wrapped by the sterilization wrapper.

3. The method as recited in claims 1 or 2, **characterized in that** the sterilization module logs a drop in temperature during sterilization below a specified value as completion of sterilization.

4. The method as recited in claims 1, 2 or 3, **characterized in that**, depending on the mode of the sterilization module, a light source on the sterilization module signals either sterility of the medical device or the alarm mode.

5. The method according to any of the preceeding claims, **characterized in that**, by their way of turning on, variously coloured light sources on the sterilization module signal the data on the efficiency of the performed sterilization and sterility of the medical device.

6. The method as recited in claims 4 or 5, **characterized in that** a light emitting diode is used as the light source.

7. The method according to any of the preceeding claims, **characterized in that** the wrapped sterilization module signals the data on the sterilization time history and sterility of the medical device to an interrogator when moved to close proximity of said interrogator.

8. The method as recited in claim 7, **characterized in that** the interrogator transfers said data on the sterilization time history to a computer.

9. The method as recited in claim 8, **characterized in that** a printer prints key data from a computer database onto a partly two-layered label, a first part of which label is stuck onto the sterilization package, which comprises the sterilized medical device and the sterilization module, and a second part of which is stuck onto a patient file prior to use of the sterilized medical device.

10. A sterilization module (SM) for controlling sterility of a sterilized medical device provided with a communication circuit, wherein it is provided with a reset circuit, which initializes and activates the sterilization module (SM)
before initiation of sterilization,
that it is provided with a data acquisition and communication circuit (DACoC) provided with at least one temperature sensor and
whereto a pressure sensor (PS) is connected,
that it is provided with a microcontroller (MCo)
connected to the data acquisition and communication circuit (DACoC) and processing the data acquired during sterilization and
shifting from a normal mode to an alarm mode
at an inefficient sterilization and/or
after a sterility expiration date of the medical device is over
and that it is provided with an acceleration sensor (AccS)
connected to an input of the microcontroller (MCo) and
triggering it for the sterilization module (SM) to signal its mode upon each jerk.

11. The sterilization module (SM) as recited in claim 10, **characterized in that** it is provided with a reset button (RB) for resetting the sterilization module (SM) to be initialized and activated hereby before the sterilization module (SM) together with the medical device is wrapped by the sterilization wrapper.

12. The sterilization module (SM) as recited in claims 10 or 1 1, **characterized in that** an output of the microcontroller (MCo) is connected to a light source (LS) that, depending on the sterilization module mode, signals either sterility of the medical device or the alarm mode.

13. The sterilization module (SM) as recited in claims 10, 1 1 or 12, **characterized in that** several outputs of the microcontroller (MCo) are connected to variously coloured light sources (LS, LS', ...) that, by the way of their turning on, signal data on the efficiency of the performed sterilization and signal an alarm mode after an inefficient sterilization and/or after a sterility expiration date of the medical device is over.

14. The sterilization module (SM) as recited in claims 1 1, 12 or 13, **characterized in that** light emitting diodes are used as light sources (LS, LS', ...).

15. The sterilization module (SM) as recited in claims 13 or 14, **characterized in that** the sterilization module (SM) transfers the processed data on the performed sterilization by means of an interrogator (In), when they are close to each other, to a personal computer (PC), wherein a base (db) of data on sterilizations is created, in which the sterilization module (SM) was used.

16. The sterilization module (SM) as recited in claims 13 or 15, **characterized in that** a two-part label is printed for the sterilized medical device, one part of which is stuck onto the sterilization package comprising the medical device and the sterilization module (SM), and a second part of which is stuck onto a patient label.

17. The sterilization module (SM) as recited in claims 15 or 16, **characterized in that** the sterilization module (SM) is fabricated as a printed circuit board (PCB) coated by a thin polymer protective coating (PcCo).

## Patentansprüche

1. Verfahren zum Kontrollieren der Sterilität einer sterilisierten medizinischen Vorrichtung nach einer Sterilisation mit Hochtemperaturdampf,
wobei die medizinische Vorrichtung in der Nähe eines Sterilisationsmoduls angeordnet wird, die beide mit einer Sterilisationsumhüllung umhüllt sind und in einen Autoklaven gegeben werden, um eine Sterilisation auszuführen, wobei
das Sterilisationsmodul zurückgesetzt wird, bevor die Sterilisation begonnen wird,
dass das Sterilisationsmodul eine Zeithistorie seiner eigenen Temperatur und seines eigenen Drucks in seiner eigenen Umgebung während der Sterilisation protokolliert,
dass das Sterilisationsmodul die Effizienz der Sterilisation durch Verarbeiten der erfassten Daten bewertet,
dass das Sterilisationsmodul von einer normalen Betriebsart nach einer ineffizienten Sterilisation und/oder nach Ablauf des Datums der Sterilität der medizinischen Vorrichtung in eine Alarmbetriebsart übergeht und
dass das Sterilisationsmodul, das mit einem Beschleunigungssensor (AccS) versehen ist, der mit einem Eingang des Mikrocontrollers (MCo) verbunden ist, seine Betriebsart bei jedem Ruck signalisiert.

2. Verfahren nach Anspruch 1, **dadurch gekennzeichnet, dass** das Sterilisationsmodul durch Drücken auf einen Rücksetzknopf zurückgesetzt wird, bevor das Sterilisationsmodul zusammen mit der medizinischen Vorrichtung mit der Sterilisationsumhüllung umhüllt wird

3. Verfahren nach den Ansprüchen 1 oder 2, **dadurch gekennzeichnet, dass** das Sterilisationsmodul einen Temperaturabfall während der Sterilisation unter einen bestimmten Wert als Abschluss der Sterilisation protokolliert.

4. Verfahren nach den Ansprüchen 1, 2 oder 3, **dadurch gekennzeichnet, dass** in Abhängigkeit von der Betriebsart des Sterilisationsmoduls eine Lichtquelle des Sterilisationsmoduls entweder die Sterilität der medizinischen Vorrichtung oder die Alarmbetriebsart signalisiert.

5. Verfahren nach einem der vorhergehenden Ansprüche, **dadurch gekennzeichnet, dass** verschiedene farbige Lichtquellen an dem Sterilisationsmodul entsprechend der Art ihres Einschaltens die Daten bezüglich der Effizienz der ausgeführten Sterilisation und die Sterilität der medizinischen Vorrichtung signalisieren.

6. Verfahren nach den Ansprüchen 4 oder 5, **dadurch gekennzeichnet, dass** als Lichtquelle eine Leuchtdiode verwendet wird.

7. Verfahren nach einem der vorhergehenden Ansprüche, **dadurch gekennzeichnet, dass** das umhüllte Sterilisationsmodul die Daten über die Sterilisationszeithistorie und die Sterilität der medizinischen Vorrichtung an eine Abfrageeinrichtung signalisiert, wenn es in die Nähe der Abfrageeinrichtung bewegt wird.

8. Verfahren nach Anspruch 7, **dadurch gekennzeichnet, dass** die Abfrageeinrichtung die Daten bezüglich der Sterilisationszeithistorie an einen Computer überträgt.

9. Verfahren nach Anspruch 8, **dadurch gekennzeichnet, dass** ein Drucker Schlüsseldaten von einer Computerdatenbank auf ein zum Teil zweischichtiges Etikett druckt, wobei ein erster Teil des Etiketts an der Sterilisationsbaugruppe, die die sterilisierte medizinische Vorrichtung und das Sterilisationsmodul umfasst, befestigt wird und ein zweiter Teil an einer Patientenakte vor der Verwendung der sterilisierten medizinischen Vorrichtung befestigt wird.

10. Sterilisationsmodul (SM) zum Kontrollieren der Sterilität einer sterilisierten medizinischen Vorrichtung, das versehen ist mit einer Kommunikationsschaltung, wobei
es mit einer Rücksetzschaltung versehen ist, die das Sterilisationsmodul (SM) vor dem Beginn der Sterilisation initialisiert und aktiviert,
dass es mit einer Datenerfassungs- und Kommunikationsschaltung (DACoC) versehen ist, die mit wenigstens einem Temperatursensor versehen ist und mit der ein Drucksensor (PS) verbunden ist,
dass es mit einem Mikrocontroller (MCo) versehen ist, der mit der Datenerfassungs- und Kommunikationsschaltung (DACoC) verbunden ist und die Daten, die während der Sterilisation erfasst werden, verarbeitet, und bei einer ineffizienten Sterilisation und/oder bei Ablauf des Datums der Sterilität der medizinischen Vorrichtung von einer normalen Betriebsart in eine Alarmbetriebsart übergeht,
und dass es mit einem Beschleunigungssensor (AccS) versehen ist, der mit einem Eingang des Mikrocontrollers (MCo) verbunden ist und diesen für das Sterilisationsmodul (SM) auslöst, um dessen Betriebsart bei jedem Ruck zu signalisieren.

11. Sterilisationsmodul (SM) nach Anspruch 10, **dadurch gekennzeichnet, dass** es mit einem Rücksetzknopf (RB) versehen ist, um das Sterilisationsmodul (SM) zurückzusetzen, das hierdurch initialisiert und aktiviert wird, bevor das Sterilisationsmodul (SM) zusammen mit der medizinischen Vorrichtung mit der Sterilisationsumhüllung umhüllt wird.

12. Sterilisationsmodul (SM) nach den Ansprüchen 10 oder 11, **dadurch gekennzeichnet, dass** ein Ausgang des Microcontrollers (MCo) mit einer Lichtquelle (LS) verbunden ist, die in Abhängigkeit von der Betriebsart des Sterilisationsmoduls entweder die Sterilität der medizinischen Vorrichtung oder die Alarmbetriebsart signalisiert.

13. Sterilisationsmodul (SM) nach den Ansprüchen 10, 11 oder 12, **dadurch gekennzeichnet, dass** mehrere Ausgänge des Microcontrollers (MCo) mit verschiedenfarbigen Lichtquellen (LS, LS', ...) verbunden sind, die entsprechend der Art ihres Einschaltens Daten bezüglich der Effizienz der ausgeführten Sterilisation signalisieren und nach einer ineffizienten Sterilisation und/oder nach Ablauf des Datums der Sterilität der medizinischen Vorrichtung eine Alarmbetriebsart signalisieren.

14. Sterilisationsmodul (SM) nach den Ansprüchen 11, 12 oder 13, **dadurch gekennzeichnet, dass** als Lichtquellen (LS, LS', ...) Leuchtdioden verwendet werden.

15. Sterilisationsmodul (SM) nach den Ansprüchen 13 oder 14, **dadurch gekennzeichnet, dass** das Sterilisationsmodul (SM) die verarbeiteten Daten für die ausgeführte Sterilisation über eine Abfrageeinrichtung (In), wenn sie sich nahe beieinander befinden, an einen Personalcomputer (PC) überträgt, wobei eine Basis (db) für Daten über die Sterilisationen erzeugt wird, bei denen das Sterilisationsmodul (SM) verwendet wurde.

16. Sterilisationsmodul (SM) nach den Ansprüchen 13 oder 15, **dadurch gekennzeichnet, dass** ein zweiteiliges Etikett für die sterilisierte medizinische Vorrichtung gedruckt wird, wovon ein Teil an der Sterilisationsbaugruppe, die die medizinische Vorrichtung und das Sterilisationsmodul (SM) enthält, und ein zweiter Teil an einem Patientenetikett befestigt werden.

17. Sterilisationsmodul (SM) nach den Ansprüchen 15 oder 16, **dadurch gekennzeichnet, dass** das Sterilisationsmodul (SM) als eine gedruckte Leiterplatte (PCB), die mit einer dünnen Polymerschutzbeschichtung beschichtet ist, hergestellt ist.

## Revendications

1. Procédé de contrôle de la stérilité d'un dispositif médical stérilisé après une stérilisation à la vapeur à haute température,
dans lequel le dispositif médical est placé à proximité d'un module de stérilisation, ils sont tous deux enveloppés par une enveloppeuse de stérilisation et mis dans un autoclave pour réaliser une stérilisation,
dans lequel
le module de stérilisation est réinitialisé avant que la stérilisation soit initiée,
que le module de stérilisation enregistre un historique de sa propre température et pression dans son propre environnement pendant la stérilisation,
que le module de stérilisation évalue l'efficacité de stérilisation en traitant les données acquises,
que le module de stérilisation passe d'un mode normal à un mode d'alarme après une stérilisation inefficace et/ou après qu'une date d'expiration de stérilité du dispositif médical a été dépassée,
et que le module de stérilisation qui est pourvu d'un capteur d'accélération (AccS) relié à une entrée du microcontrôleur (MCo) signale son mode lors de chaque secousse.

2. Procédé selon la revendication 1, **caractérisé en ce que** le module de stérilisation est réinitialisé en appuyant sur un bouton de réinitialisation avant que le module de stérilisation avec le dispositif médical soit enveloppé par l'enveloppeuse de stérilisation.

3. Procédé selon les revendications 1 ou 2, **caractérisé en ce que** le module de stérilisation enregistre une chute de température pendant la stérilisation au-dessous d'une valeur spécifiée comme la fin de la stérilisation.

4. Procédé selon les revendications 1, 2 ou 3, **caractérisé en ce que**, selon le mode du module de stérilisation, une source de lumière sur le module de stérilisation signale la stérilité du dispositif médical ou le mode d'alarme.

5. Procédé selon l'une quelconque des revendications précédentes, **caractérisé en ce que**, par leur façon de s'allumer, des sources de lumière de diverses couleurs sur le module de stérilisation signalent les données sur l'efficacité de la stérilisation effectuée et la stérilité du dispositif médical.

6. Procédé selon les revendications 4 ou 5, **caractérisé en ce qu'**une diode électroluminescente est utilisée comme source de lumière.

7. Procédé selon l'une quelconque des revendications précédentes, **caractérisé en ce que** le module de stérilisation enveloppé signale les données sur l'historique de stérilisation et la stérilité du dispositif médical à un interrogateur lorsqu'il est déplacé à proximité étroite dudit interrogateur.

8. Procédé selon la revendication 7, **caractérisé en ce que** l'interrogateur transfère lesdites donnés sur l'historique de stérilisation à un ordinateur.

9. Procédé selon la revendication 8, **caractérisé en ce qu'**une imprimante imprime des données essentielles issues d'une base de données informatique sur une étiquette partiellement à deux couches, une première partie de ladite étiquette est collée sur l'emballage de stérilisation, qui comprend le dispositif médical stérilisé et le module de stérilisation, et une deuxième partie de celle-ci est collée sur un dossier de patient avant l'utilisation du dispositif médical stérilisé.

10. Module de stérilisation (SM) destiné à contrôler la stérilité d'un dispositif médical stérilisé pourvu d'un circuit de communication,
dans lequel
il est pourvu d'un circuit de réinitialisation, qui initialise et active le module de stérilisation (SM) avant l'initialisation d'une stérilisation,
qu'il est pourvu d'un circuit d'acquisition et communication de données (DACoC) pourvu d'au moins une sonde de température et auquel un capteur de pression (PS) est relié,
qu'il est pourvu d'un microcontrôleur (MCo) relié au circuit d'acquisition et communication de données (DACoC) et traitant les données acquises pendant la stérilisation et passant d'un mode normal à un mode d'alarme lors d'une stérilisation inefficace et/ou après qu'une date d'expiration de stérilité du dispositif médical a été dépassée
et qu'il est pourvu d'un capteur d'accélération (AccS) relié à une entrée du microcontrôleur (MCo) et le déclenchant pour que le module de stérilisation (SM) signale son mode lors de chaque secousse.

11. Module de stérilisation (SM) selon la revendication 10, **caractérisé en ce qu'**il est pourvu d'un bouton de réinitialisation (RB) pour réinitialiser le module de stérilisation (SM) pour que celui-ci soit ainsi initialisé et activé avant que le module de stérilisation (SM) avec le dispositif médical soit enveloppé par l'enveloppeuse de stérilisation.

12. Module de stérilisation (SM) selon les revendications 10 ou 11, **caractérisé en ce qu'**une sortie du microcontrôleur (MCo) est reliée à une source de lumière (LS) qui, selon le mode du module de stérilisation, signale la stérilité du dispositif médical ou le mode d'alarme.

13. Module de stérilisation (SM) selon les revendications 10, 11 ou 12, **caractérisé en ce que** plusieurs sorties du microcontrôleur (MCo) sont reliées à des sources de lumière de diverses couleurs (LS, LS', ...) qui, par leur façon de s'allumer, signalent des données sur l'efficacité de la stérilisation effectuée et signalent un mode d'alarme après une stérilisation inefficace et/ou après qu'une date d'expiration de stérilité du dispositif médical a été dépassée.

14. Module de stérilisation (SM) selon les revendications 11, 12 ou 13, **caractérisé en ce que** des diodes électroluminescentes sont utilisées comme sources de lumière (LS, LS' , ...) .

15. Module de stérilisation (SM) selon les revendications 13 ou 14, **caractérisé en ce que** le module de stérilisation (SM) transfère les données traitées sur la stérilisation effectuée au moyen d'un interrogateur (In), quand ils sont proches l'un de l'autre, à un ordinateur personnel (PC), dans lequel une base de données (db) sur les stérilisations dans lesquelles le module de stérilisation (SM) a été utilisé est créée.

16. Module de stérilisation (SM) selon les revendications 13 ou 15, **caractérisé en ce qu'**une étiquette en deux parties est imprimée pour le dispositif médical stérilisé, dont une partie est collée sur l'emballage de stérilisation comprenant le dispositif médical et le module de stérilisation (SM), et dont une deuxième partie est collée sur une étiquette de patient.

17. Module de stérilisation (SM) selon les revendications 15 ou 16, **caractérisé en ce que** le module de stérilisation (SM) est fabriqué comme une carte de circuit imprimé (PCB) recouverte d'une couche mince protectrice de polymère (PcCo).
